# EUROPEAN PATENT APPLICATION

(11) **EP 3 964 226 A1**
(43) Date of publication of application: **09.03.2022**
(21) Application number: 20778386.1
(22) Date of filing: 26.03.2020
(51) Int. Cl.: A61K 38/16, A61P 37/06, A23L 33/195

(54) **COMPOSITION FOR PREVENTING OR TREATING GRAFT-VERSUS-HOST DISEASE COMPRISING TLR5 AGONIST DERIVED FROM FLAGELLIN AS EFFECTIVE COMPONENT**

(30) Priority: 28.03.2019 KR 20190035521
(71) Applicant: The Catholic University Of Korea Industry-Academic Cooperation Foundation, Seocho-gu Seoul 06591 (KR)
(72) Inventor: CHO, Seok-Goo, Seoul 06269 (KR); IM, Keon-Il, Seoul 07554 (KR); KIM, Nayoun, Seoul 05348 (KR); JEON, Young-Woo, Seoul 06630 (KR); NAM, Young-Sun, Seoul 05376 (KR); SONG, Yunejin, Changwon-si, Gyeongsangnam-do 51408 (KR); LEE, Junseok, Seoul 06788 (KR)
(74) Representative: Schnappauf, Georg
(86) International application number: PCT/KR2020/004146
(87) International publication number: WO 2020/197296

(57) **Abstract**

The present disclosure relates to a composition for preventing or treating graft-versus-host disease (GVHD) containing a TLR5 agonist derived from flagellin as an active ingredient. The TLR5 agonist derived from flagellin according to the present disclosure exhibits an excellent therapeutic effect against GVHD, and thus may be developed as an active ingredient for a composition for the treatment, prevention or alleviation of GVHD

<110>    The Catholic University of Korea Industry-Academic Cooperation
Foundation

<120>    Composition for Preventing or Treating Graft Versus Host Disease
         Comprising TLR5 Agonist Derived from Flagellin As Active
         Ingredient

<130>    DPB192042

<160>    2

<170>    KoPatentIn 3.0

<210>    1
<211>    16
<212>    PRT
<213>    Artificial Sequence

<220>
<223>    Linker Peptide


<400>    1
Ser Pro Gly Ile Ser Gly Gly Gly Gly Gly Ile Leu Asp Ser Met Gly
  1               5                  10                  15




<210>    2
<211>    295
<212>    PRT
<213>    Salmonella enterica

<400>    2
   Ala Gln Val Ile Asn Thr Asn Ser Leu Ser Leu Leu Thr Gln Asn Asn
     1               5                  10                  15
   Leu Asn Lys Ser Gln Ser Ser Leu Ser Ser Ala Ile Glu Arg Leu Ser
                20                  25                  30
   Ser Gly Leu Arg Ile Asn Ser Ala Lys Asp Asp Ala Ala Gly Gln Ala
            35                  40                  45
   Ile Ala Asn Arg Phe Thr Ser Asn Ile Lys Gly Leu Thr Gln Ala Ser
        50                  55                  60
   Arg Asn Ala Asn Asp Gly Ile Ser Ile Ala Gln Thr Thr Glu Gly Ala
    65                  70                  75                  80
   Leu Asn Glu Ile Asn Asn Asn Leu Gln Arg Val Arg Glu Leu Ser Val
                    85                  90                  95
   Gln Ala Thr Asn Gly Thr Asn Ser Asp Ser Asp Leu Lys Ser Ile Gln
               100                 105                 110
   Asp Glu Ile Gln Gln Arg Leu Glu Glu Ile Asp Arg Val Ser Asn Gln
           115                 120                 125
   Thr Gln Phe Asn Gly Val Lys Val Leu Ser Gln Asp Asn Gln Met Lys
       130                 135                 140
   Ile Gln Val Gly Ala Asn Asp Gly Glu Thr Ile Thr Ile Asp Leu Gln
   145                 150                 155                 160
   Lys Ile Asp Val Lys Ser Leu Gly Leu Asp Gly Phe Asn Val Asn Ser
                   165                 170                 175
   Pro Gly Ile Ser Gly Gly Gly Gly Gly Ile Leu Asp Ser Met Gly Thr
               180                 185                 190
   Leu Ile Asn Glu Asp Ala Ala Ala Ala Lys Lys Ser Thr Ala Asn Pro
           195                 200                 205
   Leu Ala Ser Ile Asp Ser Ala Leu Ser Lys Val Asp Ala Val Arg Ser
       210                 215                 220
   Ser Leu Gly Ala Ile Gln Asn Arg Phe Asp Ser Ala Ile Thr Asn Leu
   225                 230                 235                 240
   Gly Asn Thr Val Thr Asn Leu Asn Ser Ala Arg Ser Arg Ile Glu Asp
                   245                 250                 255
   Ala Asp Tyr Ala Thr Glu Val Ser Asn Met Ser Lys Ala Gln Ile Leu
               260                 265                 270
   Gln Gln Ala Gly Thr Ser Val Leu Ala Gln Ala Asn Gln Val Pro Gln
           275                 280                 285
   Asn Val Leu Ser Leu Leu Arg
       290                 295

## Description

### Technical Field

The present disclosure relates to a composition for preventing or treating graft-versus-host disease containing, as an active ingredient, a TLR5 agonist derived from flagellin.

### Background Art

Toll-like receptor 5 (TLR5) is a protein encoded by the TLR5 gene in humans (PNAS. 95 (2): 588-93), and is a member of the toll-like receptor (TLR) family. TLR5 is known to be able to recognize flagellin from invading motile bacteria (Seminars in Immunopathology. 29 (3): 275-88). TLR5 is known to be involved in the onset of various diseases, including inflammatory bowel disease (Journal of Physiology and Pharmacology. 60 Suppl 4: 71-5).

Flagellin is a major structural protein constituting bacterial flagellar filaments which are mobile cell organelles. Tens of thousands of flagellin molecules polymerize helically to form long whip-like flagellar filaments. Flagellin includes a D0 domain, a D1 domain, a D2 domain, and a D3 domain. Among them, the D0 domain and the D1 domain are required for filament assembly. The D0 and D1 domains of flagellin are highly conserved in structure and sequence among a variety of bacterial species and function as common molecular patterns of bacteria having flagella to alert the host of bacterial infection. It is known that flagellin is recognized by TLR5 and activates the NF-κB signaling mechanism to induce innate immune stimulation, cytoprotection and radioresistance.

Graft-versus-host disease (GVHD) occurs in the process of transplanting allogeneic hematopoietic stem cells for the treatment of blood cancers such as leukemia, myeloma, lymphoma and aplastic anemia. It occurs when lymphocytes transfused through transplantation or blood transfusion attack an immunocompromised host. Transfused lymphocytes are generally destroyed by the host's immune mechanism, and when the host's immune function is compromised, the host's immune system cannot perform this function, and thus GVHD occurs. Meanwhile, the higher the degree of immunosuppression of the host, the higher the possibility of GVHD. Graft-versus-host disease and the graft-versus-tumor (GVT) effect are closely related to each other, and if the degree of immunosuppression after transplantation is increased, the incidence of GVHD is reduced, but the GVT effect is also weakened, resulting in an increase in the recurrence of leukemia. Therefore, there is an urgent need to develop a new therapeutic agent capable of maximizing the GVT effect while inhibiting GVHD

The patent documents and references mentioned in this specification are incorporated herein by reference to the same extent as if each reference was individually and specifically indicated to be incorporated by reference.

### DISCLOSURE

### Technical Problem

The present inventors have researched and endeavored to develop a therapeutic agent capable of maximizing the graft-versus-tumor (GVT) effect while effectively inhibiting graft-versus-host disease (GVHD). As a result, the present inventors have developed a novel peptide agonist of toll-like receptor 5 (TLR5) derived from flagellin, and have experimentally found that this TLR5 agonist effectively inhibits graft-versus-host disease, thereby completing the present disclosure.

Therefore, an object of the present disclosure is to provide a pharmaceutical composition for treating or preventing graft-versus-host disease containing: a therapeutically effective amount of a TLR5 agonist derived from flagellin; and a pharmaceutically acceptable carrier.

Another object of the present disclosure is to provide a functional food composition for alleviating graft-versus-host disease containing, as an active ingredient, the TLR5 agonist derived from flagellin.

Other objects and technical features of the present disclosure will be more clearly understood from the following detailed description of the present disclosure, the appended claims and the accompanying drawings.

### Technical Solution

In accordance with one aspect of the present disclosure, there is provided a pharmaceutical composition for treating or preventing graft-versus-host disease containing: (i) a therapeutically effective amount of a TLR5 agonist derived from flagellin; and (ii) a pharmaceutically acceptable carrier.

As used herein, the term "TLR5 agonist derived from flagellin" is meant to include all proteins or polypeptides derived from bacterial flagellin protein or obtained by modifying the same, which have an activity of activating Toll-like receptor 5 (TLR5)-mediated signaling.

As used herein, the term "flagellin" refers to a major protein constituting bacterial flagellar filaments. Flagellin includes a D0 domain, a D1 domain, a D2 domain, and a D3 domain. It is known that flagellin is recognized by TLR5 and activates the NF-κB signaling mechanism to induce innate immune stimulation, cytoprotection and radioresistance.

According to one embodiment of the present disclosure, the "TLR5 agonist derived from flagellin" may be a peptide substance including the D0 domain of flagellin and the D1 domain of flagellin.

According to another embodiment of the present disclosure, the "TLR5 agonist derived from flagellin" may include the D0 domain of flagellin, the D1 domain of flagellin, and a linker peptide.

According to still another embodiment of the present disclosure, the linker peptide may be included within the D1 domain.

According to yet another embodiment of the present disclosure, the linker peptide may include the amino acid sequence of SEQ ID NO: 1.

According to still yet another embodiment of the present disclosure, the "TLR5 agonist derived from flagellin" includes the amino acid sequence of SEQ ID NO: 2.

"Graft-versus-host disease (GVHD)", a disease to be treated in the present disclosure, is a systemic disease which occurs when transfused lymphocytes attack a host with compromised immune function, and in which T cells from a donor damage the host organs, for example, liver, small intestine, large intestine, skin, and the like. Transfused lymphocytes are generally destroyed by the host's immune mechanism, and when the host's immune function is compromised, the host's immune system cannot perform this function, and thus GVHD occurs. The higher the degree of immunosuppression, the higher the possibility of GVHD due to transfusion. GVHD appears in patients who inevitably require hematopoietic stem cell transplantation due to malignant blood cancer (acute leukemia or Hodgkin's disease, etc.) or congenital immunodeficiency, and the higher the complicated immunosuppression, the higher the possibility of graft-versus-host disease (GVHD). Graft-versus-host disease (GVHD) occurring after bone marrow transplantation is classified into two types, acute and chronic, and graft-versus-host disease occurring after transfusion is acute. Symptoms thereof include fever, rash, itching, jaundice, liver dysfunction, diarrhea, bleeding, pancytopenia (a state in which all white blood cells, red blood cells, and platelets are reduced), etc., and occur between 4 and 30 days after transfusion.

Meanwhile, a graft-versus-tumor is an immune response to recipient's cancer tissue by donor's immune cells present in the transplanted bone marrow or tissue. In bone marrow transplantation, etc., when the compatibility between the donor and the recipient is not suitable, the transplanted leukocytes attack the normal somatic cells of the recipient, causing graft-versus-host disease. An immunosuppressant is administered to inhibit graft-versus-host disease (GVHD) which is an immune disorder, but it may cause recurrence of leukemia by inevitably lowering the graft-versus-tumor (GVT) effect that attacks cancer cells. Thus, it can be said that graft-versus-host disease and the graft-versus-tumor (GVT) effect are closely related to each other.

As demonstrated by the experimental results in the Examples of the present specification, the TLR5 agonist derived from flagellin according to the present disclosure showed an excellent therapeutic effect against GVHD in a GVHD-induced animal model.

In addition, as demonstrated by other experimental results in the Examples of the present specification, the TLR5 agonist derived from flagellin according to the present disclosure showed the effect of promoting the production of host-derived IL (interleukin)-22 and IL-23. Thus, the flagellin-derived TLR5 agonist of the present disclosure may promote the production of host-derived IL-22 and IL-23, thereby suppressing a decrease in intestinal stem cells in a host suffering from graft-versus-host disease.

As demonstrated by the experimental results in the Examples of the present specification, the flagellin-derived TLR5 agonist of the present disclosure showed an excellent effect of increasing the graft-versus-tumor effect in a GVHD- and GVT-induced animal model.

The composition of the present disclosure may be provided as a pharmaceutical composition containing: (i) a therapeutically effective amount of a TLR5 agonist derived from flagellin; and (ii) a pharmaceutically acceptable carrier.

As used herein, the term "therapeutically effective amount" refers to an amount suitable for treating or preventing graft-versus-host disease by administering the "TLR5 agonist derived from flagellin" as an active ingredient, to a subject patient. Specifically, the term "therapeutically effective amount" refers to a sufficient amount of an agent or compound being administered which will relieve to some extent one or more of the symptoms of the disease or condition being treated.

The preferred dosage of the pharmaceutical composition containing: (i) a therapeutically effective amount of a TLR5 agonist derived from flagellin; and (ii) a pharmaceutically acceptable carrier, may be suitably adjusted. Preferably, the daily dosage of the composition may be 25 to 100 µg/kg.

The pharmaceutical composition of the present disclosure may contain a pharmaceutically acceptable carrier, in addition to the "TLR5 agonist derived from flagellin" as an active ingredient. Examples of this carrier include, but are not limited to, lactose, dextrose, sucrose, sorbitol, mannitol, starch, gum acacia, calcium phosphate, alginate, gelatin, calcium silicate, microcrystalline cellulose, polyvinyl pyrrolidone, cellulose, water, syrup, methyl cellulose, methylhydroxybenzoate, propylhydroxybenzoate, talc, magnesium stearate, menthol and mineral oil, which are commonly used in formulation.

The pharmaceutical composition of the present disclosure may further contain a lubricant, a wetting agent, a sweetening agent, a flavoring agent, an emulsifying agent, a suspending agent, a preservative, and the like, in addition to the above-described ingredients. Suitable pharmaceutically acceptable carriers and agents are described in detail in Remington's Pharmaceutical Sciences (19th ed., 1995).

A suitable dosage of the pharmaceutical composition of the present disclosure may vary depending on factors such as formulation method, administration mode, the patient's age, weight, sex, disease condition and diet, administration time, administration route, excretion rate, and response sensitivity.

The pharmaceutical composition of the present disclosure may be administered orally or parenterally. When the pharmaceutical composition is administered parenterally, it may be administered by intravenous injection, subcutaneous injection, intramuscular injection, intraperitoneal injection, transdermal administration, or the like.

Meanwhile, since the pharmaceutical composition of the present disclosure is applied for the treatment or prevention of graft-versus-host disease, the pharmaceutical composition of the present disclosure may be administered by various oral or parenteral routes.

The concentration of the active ingredient contained in the pharmaceutical composition of the present disclosure may be determined in consideration of the purpose of treatment, the condition of the patient, the required period, and the like, and is not limited to a concentration within a specific range.

The pharmaceutical composition of the present disclosure may be prepared in a unit dosage form by formulation using a pharmaceutically acceptable carrier and/or excipient according to a method that may be easily performed by a person of ordinary skill in the art to which the present invention pertains, or may be provided in a multiple-dose container. At this time, the formulation may be in the form of a solution, suspension or emulsion in oil or aqueous medium, or may be in the form of an extract, powder, granules, a tablet or a capsule, and may additionally contain a dispersant or stabilizer.

According to another aspect of the present disclosure, the present disclosure provides a functional food composition for alleviating graft-versus-host disease containing, as active ingredient, a TLR5 agonist derived from flagellin.

The functional food composition of the present disclosure contains ingredients which are commonly added during food production, and contains, for example, protein, carbohydrate, fat, a nutrient and a flavoring agent. For example, the functional food composition may contain a flavoring agent or natural carbohydrate as an additional ingredient, in addition to the TLR5 agonist derived from flagellin as an active ingredient. Examples of the natural carbohydrate include monosaccharides (e.g., glucose, fructose, etc.); disaccharides (e.g., maltose, sucrose, etc.); oligosaccharides; polysaccharides (e.g., dextrin, cyclodextrin, etc.); and sugar alcohols (e.g., xylitol, sorbitol, erythritol, etc.). Examples of the flavoring agent include natural flavoring agents (e.g., thaumatin, stevia extract, etc.) and synthetic flavoring agents (e.g., saccharin, aspartame, etc.).

The advantages of the present disclosure are summarized as follows:
(i) The TLR5 agonist derived from flagellin according to the present disclosure shows an excellent therapeutic effect against graft-versus-host disease (GVHD).
(ii) The TLR5 agonist derived from flagellin according to the present disclosure may be developed as an active ingredient for a composition for treating, preventing or alleviating graft-versus-host disease.

### Advantageous Effects

The present disclosure relates to a composition for preventing or treating graft-versus-host disease containing, as an active ingredient, a TLR5 agonist derived from flagellin. The TLR5 agonist derived from flagellin according to the present disclosure shows an excellent therapeutic effect against graft-versus-host disease (GVHD), and thus may be developed as an active ingredient for a composition for treating, preventing or alleviating graft-versus-host disease.

### Brief Description of Drawings

FIG. 1 depicts experimental result photographs showing the GVHD inhibitory effect of administration of a TLR5 agonist, which is a polypeptide having the amino acid sequence of SEQ ID NO: 2 (hereinafter, the TLR5 agonist of SEQ ID NO: 2 is referred to as "KMRC011"), in a GVHD mouse animal model. The GVHD mouse animal model was obtained using C57BL/6 (H-2kb) mice (donor) and BALB/c (H-2kd) mice (recipient) with different MHC classes by transplanting bone marrow cells and splenocytes of the donor into the vein of the recipient.
FIG. 2 shows experimental results showing the GVHD inhibitory effect of KMRC011 in the GVHD mouse animal model. The graphs show the results of measuring the survival rate, body weight, and clinical GVHD score of the GVHD mouse model.
FIG. 3 depicts immunohistochemical staining images showing a histological difference and the GVHD inhibitory effect of KMRC011 administration in the GVHD mouse model.
FIG. 4 depicts results showing the GVHD inhibitory effect of KMRC011 in the GVHD mouse model, and shows the effects of KMRC011 administration on increased expression of IL-22 and IL-23 and the protection of intestinal stem cells.
FIG. 5 shows the result of measuring the biological activities of KMRC011 and flagellin in HEK-Dual cells generated by transfection with the TLR5 gene, and shows the results of detecting IL-8 response using a luminometer.
FIG. 6 shows the results of measuring the biological activities of KMRC011 and flagellin in HEK-Dual cells generated by transfection with the TLR5 gene, and shows the results of detecting NF-κB response by an alkaline phosphatase activity (AP) reaction measurement method.
FIG. 7 depicts results showing the GVHD inhibitory effect of KMRC011 administration in a GVHD and GVT mouse model.
FIG. 8 depicts results showing an increased GVT effect induced by KMRC011 administration in the GVHD and GVT mouse model, and shows the results of tracking and observing tumors using an *in vivo* fluorescence imaging system (IVIS Lumina XRMS).

### Mode for Invention

The specific examples described in the present specification are representatives of preferred examples of the present disclosure, and the scope of the present disclosure is not limited thereto. It will be apparent to those skilled in the art that modifications and other uses of the present disclosure do not depart from the scope of the present disclosure disclosed in the appended claims.

### Examples

### Experimental Methods

### 1. Mouse Model Establishment

### (1) GVHD Mouse Model

6-8-week-old C57BL/6 (H-2kb) and BALB/c (H-2kd) mice with different MHC classes were used. The recipient mouse BALB/c (H-2kd) was systemically irradiated with 800 cGy, and within 24 hours, bone marrow cells (5 × 10⁶ cells) and splenocytes (5 × 10⁶ cells) of the donor mouse C57BL/6 (H-2kb) were isolated and transplanted into the vein of the allogeneic recipient mouse to induce a GVHD animal model. Then, the GVHD mice were observed and evaluated for their weight, posture, activity, hair condition and skin density twice a week according to the clinical GVHD scoring system (perfect score: 10, score for each item: 2; see Table 1 below). Table 1 below describes clinical evaluation criteria for the weight, posture, activity, hair condition and skin density of the GVHD mouse model.

**[Table 1]**

| Score | Weight | Posture | Activity | Hair condition | Skin density |
|---|---|---|---|---|---|
| 0 | <10% | Normal | Normal | Normal | Normal |
| 1 | >10% to <25% | Slightly hunching | Slightly inactive | Mild ruffling | Scaling of paws and tail |
| 2 | >25% | Severely hunching | Not response to stimulus and inactive | Severe ruffling | Obviously denuded |

### (2) GVHD and GVT Mouse Model

In order to simultaneously verify the effectiveness of a GVHD treating agent in tumor patients by confirming the effects of KMRC011 on the migration pathway, survival rate and proliferation rate of tumor cells and on antitumor effects by introducing a molecular imaging technique, a model in which both GVHD and GVT were induced was established by allogeneic hematopoietic stem cell transplantation into tumor-bearing mice.

A cancer cell line (A20 1×10⁶) whose tumor can be tracked by molecular labeling was intravenously injected into mice, and after 7 days, the mice were irradiated with radiation (8 Gy) and subjected to allotransplantation, thereby establishing a GVT model. The whole body of each animal injected with luciferin was imaged using an *in vivo* fluorescence imaging system (IVIS Lumina XRMS) in a living state, and GVHD assessment was performed.

### 2. Immunohistochemistry

4 µm-serial sections were obtained from paraffin-embedded tissue, and treated three times with xylene to remove paraffin, and then hydrated sequentially in 95%, 90% and 70% ethanol. Then, the sections were immersed in 0.5% hydrogen peroxide to remove endogenous peroxidase. Then, the sections were treated with normal goat serum for 30 minutes and incubated for 1 hour with a primary antibody diluted in phosphate buffered saline (PBS) containing 3% BSA (bovine serum albumin) according to the manufacturer's instruction. After the primary antibody response to the protein encoded by the gene showing a difference in expression, the sections were washed three times with Tris-buffer saline (TBS) and incubated for 30 minutes with 5 µg/ml of biotinylated anti-mouse/anti-rabbit IgG diluted in 3% BSA. Next, the sections were incubated with 3 µg/ml of horseradish peroxidase streptavidin for 30 minutes, color-developed using diaminobenzidine (DAB) and hydrogen peroxide, and then counterstained with Meyer's hematoxylin or 1% methyl green.

### 3. HEK-Dual ^{™} hTLR5 (NF / IL8) Cell Culture

As a TLR5 agonist, a polypeptide ("KMRC011") having the amino acid sequence of SEQ ID NO: 2 was used. In order to compare the biological activities of KMRC011 of the present disclosure and flagellin that respond to TLR5, HEK-Dual^{™} hTLR5 (NF/IL8) cells were used. HEK-Dual^{™} cells are cells that can be used to study only hTLR5 signaling by stable transfection with human TLR5 (hTLR5) gene and knockout of TLR3 and TNFR without interference of other TLRs and cytokines TNF-a, and were obtained from InvivoGen. These cells stably express a SEAP (secreted embryonic alkaline phosphatase) reporter structure capable of inducing NF-κB/AP-1 by TLR5 signaling, and express Lucia luciferase under the control of an endogenous IL-8 promoter. The cells were cultured in a DMEM medium (Life Technologies) containing 10% FBS, 50 U/ml penicillin, 50 µg/ml streptomycin, 100 µg/ml Normocin^{™}, 100 µg/ml Hygromycin B Gold and 50 µg/ml Zeocin^{™} under conditions of 5% CO₂ and 37°C. Each of the TLR5 agonist (KMRC011) of the present disclosure and flagellin was diluted to 100 ng/ml, 10 ng/ml, 1 ng/ml, 0.1 ng/ml and 0.01ng/ml, and 20 µl of each dilution was added to each well of a flat-bottom 96-well plate. To another well, 20 µl of sterile water as a negative control was added. A HEK-Dual^{™} hTLR5 (NF / IL8) cell suspension was prepared, and then 180 µl (10,000 cells) of the cell suspension was added to each well of the flat-bottom 96-well plate and incubated for 20 to 24 hours under conditions of 5% CO₂ and 37°C.

### 4. Detection of IL-8 response in HEK-Dual^{™} hTLR5 cells

QUANTI -Luc^{™} is an assay reagent capable of quantitatively measuring the activity of Lucia luciferase since it contains all components such as a stabilized substrate required for the luciferase reaction. Luminescence produced by the luciferase reaction can be quantified using a luminometer. 10 µl of the HEK-Dual^{™} hTLR5 (NF-κB / IL8) cell culture supernatant was dispensed into each well of a flat-bottom 96-well plate, and then 50 µl of QUANTI-Luc^{™} was added thereto, and then immediately, the luminescence of each well was measured using a luminometer.

### 5. Detection of NF-κB Response in HEK-Dual^{™} hTLR5 Cells

QUANTI-Blue^{™} is a colorimetric enzyme assay reagent developed to measure alkaline phosphatase (AP) activity in biological samples such as cell culture supernatants, and changes from pink to purple-blue in the presence of AP. 180 µl of QUANTI-Blue^{™} (InvivoGen) was dispensed into each well of a flat-bottom 96-well plate, and then 20 µl of the induced HEK-Dual^{™} hTLR5 (NF-κB/IL8) cell culture supernatant was added to each well. Next, each well was incubated at room temperature for 5 to 30 minutes, and then the SEAP level was measured at 620 to 655 nm using a spectrophotometer.

### Experimental Results

### 1. Verification of GVHD inhibitory effect of TLR5 agonist in GVHD mouse model

After induction of the GVHD mouse model, the GVHD inhibitory effect of the TLR5 agonist of the present disclosure ("KMRC011") was observed. Detailed methods for administration of drugs were as follows. Recipient mice were pretreated, and each of KMRC011 25 µg/kg (V), KMRC011 50 µg/kg (◊), KMRC011 100 µg/kg (△) and vehicle (control group; ■) was intraperitoneally injected into the mice a total of 5 times at 2-day intervals.

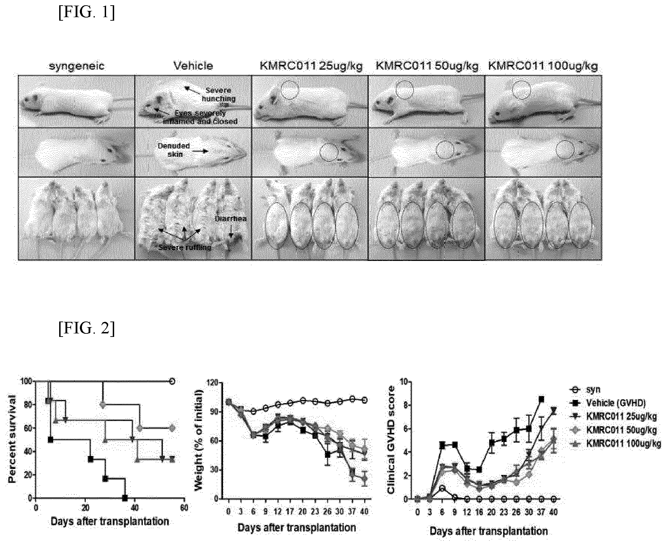

The mice of each group were earmarked and evaluated for their weight, posture, activity, hair condition and skin density twice a week. As a result of the evaluation experiment, as shown in FIGS. 1 and 2, the vehicle group in the GVHD mouse model showed weight loss, back hunching, decreased activity, and changes in the appearance of hair and skin strength. However, it could be confirmed that the above symptoms were significantly reduced in the KMRC011-administered group. In addition, it was confirmed that, in the KMRC011-administered group, the survival rate significantly increased compared to that in the vehicle group, and the symptoms in clinical GVHD scores significantly decreased.

### 2. Histological evaluation after administration of TLR5 agonist in GVHD mouse model

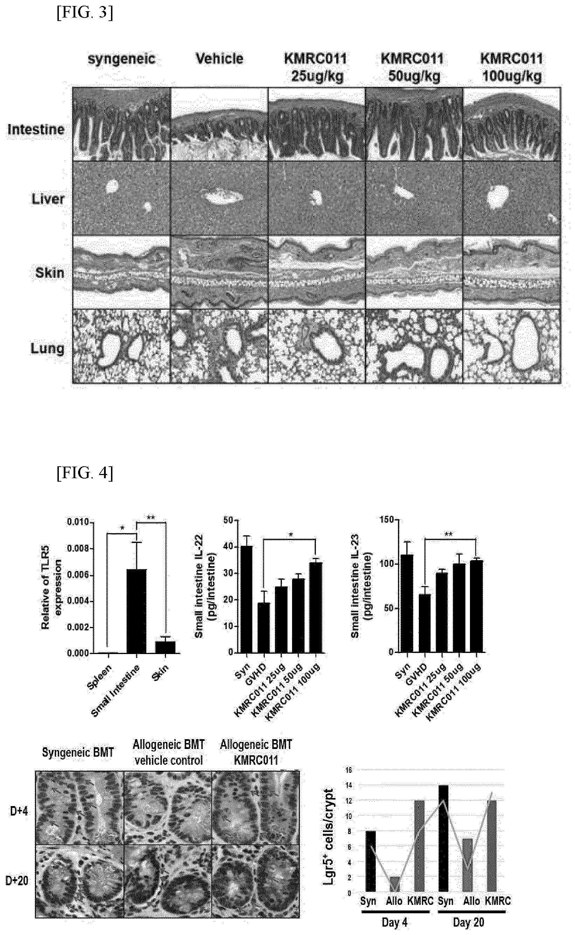

After KMRC011 was administered to the GVHD mouse model, the intestine, liver, skin and lung tissues of the animal model were isolated and fixed in 4% formaldehyde, and then paraffin blocks were prepared therefrom and histological findings were analyzed by H&E staining. As a result, as shown in FIG. 3, it was confirmed that, in the vehicle group, the villi and mucous membranes in the intestine were destroyed and infiltration of inflammatory cells in the liver and skin was observed, whereas, in the KMRC011-administerd group, findings similar to normal histological findings appeared.

### 3. Effects of TLR5 agonist administration on increased expression of IL-22 and IL-23 and protection of intestinal stem cells in GVHD mouse model

After administration of KMRC011 to the GVHD mouse model, the effects of KMRC011 on the expression levels of IL-22 and IL-23 and the protection of intestinal stem cells were evaluated. As shown in FIG. 4, the expression of TLR5 was highest in the small intestine than in the spleen and skin, and when KMRC011 was administered to the GVHD mouse model, the expression of IL-22 and IL-23 in the intestine significantly increased. In addition, it was observed that, in the GVHD animal model, a decrease in intestinal Lgr5+ stem cells was observed, but in the KMRC011-administered group in the GVHD animal model, Lgr5+ stem cells were effectively protected (see FIG. 4).

### 4. Results of Comparison between TLR5 response activities of TLR5 agonist of the present disclosure and flagellin

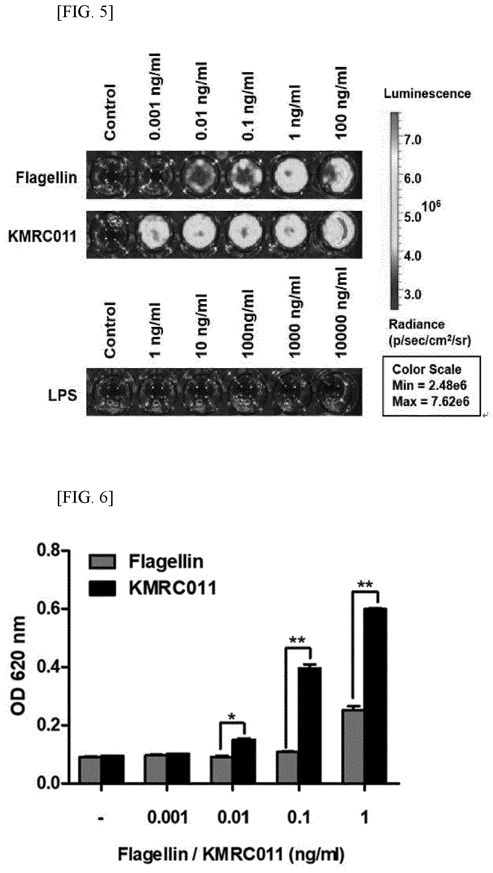

The TLR5 activation effects of KMRC011 and flagellin in HEK-Dual cells generated by transfection with TLR5 gene were compared with each other. IL-8 response was detected with a luminometer using HEK-293 reporter cells. As a result, as shown in FIG. 5, at a low concentration (0.001 to 0.01 ng/ml), the activation response of KMRC011 was much higher than that of flagellin. On the other hand, when treated with LPS, which is a negative control, biological activity of TLR5 was not observed (FIG. 5). In addition, NF-κB response was detected by an alkaline phosphatase activity (AP) measurement method under the same conditions. As a result, as shown in FIG. 6, it was confirmed that the reactivity of KMRC011 was higher than that of flagellin at a concentration of 0.01 nm/ml or higher.

### 5. Verification of effect of TLR5 agonist on GVHD Inhibition and GVT increase in GVHD and GVT mouse model

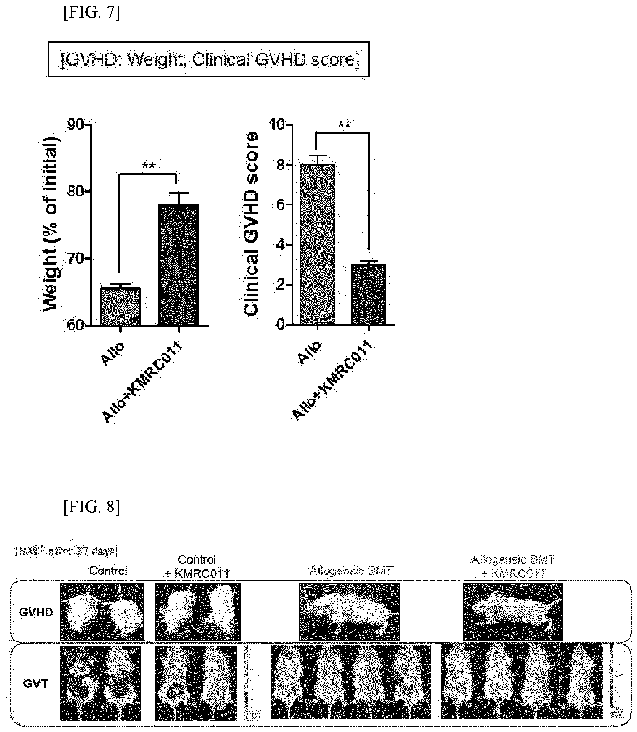

The effect of KMRC011 administration on both the inhibition of GVHD and the maximization of the GVT effect was verified. Three hours before irradiation, 50 µg/kg of KMRC011 was intraperitoneally administered once to the GVHD and GVT mouse model which is a tumor-bearing animal model. It could be confirmed that the group to which KMRC011 (allogeneic BMT group) was not administered showed weight loss, back hunching, decreased activity, and changes in the appearance of hair and skin strength, suggesting that GVHD occurred, whereas, in the group (allogeneic BMT+KMRC011 group) to which KMRC011 was administered, the above symptoms significantly decreased (see FIG. 7).

Additionally, tumors were tracked and observed using an *in vivo* fluorescence imaging system (IVIS Lumina XRMS). Some tumors were found in the allogeneic BMT group, whereas no tumors were found in the allogeneic BMT+KMRC011 group. It was demonstrated that KMRC011 is an innovative therapeutic agent capable of increasing the GVT effect while inhibiting GVHD (see FIG. 8).

### Industrial Applicability

The present disclosure relates to a composition for preventing or treating graft-versus-host disease containing, as an active ingredient, a TLR5 agonist derived from flagellin. The TLR5 agonist derived from flagellin according to the present disclosure exhibits an excellent therapeutic effect against graft-versus-host disease (GVHD).

## Claims

1. A pharmaceutical composition for treating or preventing graft-versus-host disease containing: (i) a therapeutically effective amount of a TLR5 agonist derived from flagellin; and (ii) a pharmaceutically acceptable carrier.

2. The pharmaceutical composition of claim 1, wherein the TLR5 agonist derived from flagellin comprises the D0 domain and D1 domain of flagellin.

3. The pharmaceutical composition of claim 1, wherein the TLR5 agonist derived from flagellin comprises a linker peptide, which has the amino acid sequence set forth in SEQ ID NO: 1, within the D1 domain.

4. The pharmaceutical composition of claim 1, wherein the TLR5 agonist derived from flagellin comprises the amino acid sequence set forth in SEQ ID NO: 2.

5. The pharmaceutical composition of claim 1, wherein the TLR5 agonist derived from flagellin promotes production of IL-22 and IL-23 derived from a host.

6. The pharmaceutical composition of claim 1, wherein the TLR5 agonist derived from flagellin suppresses a decrease in intestinal stem cells of a host.

7. The pharmaceutical composition of claim 1, which increases graft-versus-tumor effect.

8. A functional food composition for alleviating graft-versus-host disease containing, as an active ingredient, a TLR5 agonist derived from flagellin.

9. The functional food composition of claim 8, wherein the TLR5 agonist derived from flagellin comprises the D0 domain and D1 domain of flagellin.

10. The functional food composition of claim 8, wherein the TLR5 agonist derived from flagellin comprises a linker peptide having the amino acid sequence set forth in SEQ ID NO: 1.

11. The functional food composition of claim 8, wherein the TLR5 agonist derived from flagellin comprises the amino acid sequence set forth in SEQ ID NO: 2.

12. The functional food composition of claim 8, which increases graft-versus-tumor effect.
